# EUROPEAN PATENT SPECIFICATION

(11) **EP 1 879 029 B1**
(45) Date of publication and mention of the grant of the patent: **15.12.2010**
(21) Application number: 05745128.8
(22) Date of filing: 30.04.2005
(51) Int. Cl.: G01N 33/53, G01N 33/535, G01N 33/577, G01N 33/68, G01N 33/72

(54) **A NEUROGLOBIN ENZYME-LINKED IMMUNODETECTION KIT AND THE USE OF IT**
IMMUNNACHWEISKIT IN VERBINDUNG MIT NEUROGLOBIN-ENZYM SOWIE VERWENDUNG DAVON
NECESSAIRE DE DETECTION IMMUNOLOGIQUE D UNE NEUROGLOBINE LIEE A UNE ENZYME ET SON UTILISATION

(43) Date of publication of application: 16.01.2008
(73) Proprietor: Institute of Radiation Medicine, Academy of Military Medical Sciences; People's Liberation Army of China, Haidan District Beijing 100850 (CN); Beijing Threeman Medicine Science and Technology Limited Company, Haidan District Beijing 100856 (CN)
(72) Inventor: WANG, Lihong, Haidian District, Beijing 100850 (CN); CHEN, Tingfang, Haidian District, Beijing 100850 (CN); SHANG, Aijia, Haidian District, Beijing 100850 (CN); GAO, Yan, Haidian District, Beijing 100850 (CN); ZHANG, Chennang, Haidian District, Beijing 100850 (CN)
(74) Representative: von Kreisler Selting Werner
(86) International application number: PCT/CN2005/000613
(87) International publication number: WO 2006/116898

(56) References cited:
- WO-A-03/040332
- US-A1- 2005 069 557
- WYSTUB SYLVIA ET AL: "Localization of neuroglobin protein in the mouse brain." NEUROSCIENCE LETTERS, vol. 346, no. 1-2, 31 July 2003 (2003-07-31), pages 114-116, XP002506863 ISSN: 0304-3940
- GROZDANIC S D ET AL: "Neuroglobin and Histoglobin (cytoglobin) - New hexacoordinate globins in the human eye" IOVS, vol. 45, no. Suppl. 1, April 2004 (2004-04), page U967, XP002506864 & ANNUAL MEETING OF THE ASSOCIATION-FOR-RESEARCH-IN-VISION-AND-OPH THALM OLOGY; FT LAUDERDALE, FL, USA; APRIL 24 -29, 2004 ISSN: 0146-0404
- SUN Y ET AL: "Neuroglobin is up-regulated by and protects neurons from hypoxic-ischemic injury" PROCEEDINGS OF THE NATIONAL ACADEMY OF SCIENCES OF THE UNITED STATES OF AMERICA 20011218 US, vol. 98, no. 26, 18 December 2001 (2001-12-18), pages 15306-15311, XP002506865 ISSN: 0027-8424 Retrieved from the Internet: URL:http://abstracts.iovs.org/cgi/content/ abstract/45/5/2586> [retrieved on 2008-12-04]
- OSTERGAARD P B ET AL: "An enzyme linked immunosorption assay for tissue factor pathway inhibitor." THROMBOSIS RESEARCH 1 SEP 1997, vol. 87, no. 5, 1 September 1997 (1997-09-01), pages 447-459, XP002506866 ISSN: 0049-3848
- SAGAWA K ET AL: "Detection and quantitation of a sodium-dependent sulfate cotransporter (NaSi-1) by sandwich-type enzyme-linked immunosorbent assay" PFLUGERS ARCHIV EUROPEAN JOURNAL OF PHYSIOLOGY 1998 DE, vol. 437, no. 1, 1998, pages 123-129, XP002506867 ISSN: 0031-6768
- HUUSKONEN J ET AL: "Quantification of human plasma phospholipid transfer protein (PLTP): Relationship between PLTP mass and phospholipid transfer activity" ATHEROSCLEROSIS 200008 IE, vol. 151, no. 2, August 2000 (2000-08), pages 451-461, XP002506868 ISSN: 0021-9150
- ZHANG C. ET AL.: 'Prokaryotic Expression, Antibody Preparation and Cellular Distribution of Rat Neuroglobin' CHINESE JOURNAL OF BIOCHEMISTRY AND MOLECULAR BIOLOGY vol. 18, no. 1, February 2002, pages 80 - 84, XP008115228
- SUN J. ET AL.: 'Prokaryoti Soluble Expression Preparation and Characterization of Monoclonal Antibody of Rat Neuroglobin' CHINESE JOURNAL OF BIOCHEMISTRY AND MOLECULAR BIOLOGY vol. 19, no. 6, December 2003, pages 751 - 756, XP008115229
- DENG M. ET AL.: 'Immunohistochemical Study on the distribution of NGB proteins in the central nervous system of adult rats' CHINESE JOUNAL OF HISTOCHEMISTRY AND CYTOCHEMISTRY vol. 11, no. 3, September 2002, pages 271 - 274, XP008115231
- ZHANG C. ET AL.: 'Coding Region cDNA Sequence Cloning of Rat Neuroglobin Gene, Its Polymorphism Feature and Tissue Expression Profile Analysis' ACTA GENETICA SINICA vol. 28, no. 11, 2001, pages 997 - 1001, XP008115230
- CHAO Y-P ET AL: "HIGH PRODUCTION OF HETEROLOGOUS PROTEINS IN ESCHERICHIA COLI USING THE THERMO-REGULATED T7 EXPRESSION SYSTEM" APPLIED MICROBIOLOGY AND BIOTECHNOLOGY, SPRINGER VERLAG, BERLIN, DE, vol. 58, no. 4, 1 March 2002 (2002-03-01), pages 446-453, XP009069009 ISSN: 0175-7598
- OU J ET AL: "Stationary phase protein overproduction is a fundamental capability of Escherichia coli" BIOCHEMICAL AND BIOPHYSICAL RESEARCH COMMUNICATIONS, ACADEMIC PRESS INC. ORLANDO, FL, US, vol. 314, no. 1, 30 January 2004 (2004-01-30), pages 174-180, XP004483577 ISSN: 0006-291X
- WANG L ET AL: "Functional expression and characterization of four novel neurotoxins from sea anemone Anthopleura sp" BIOCHEMICAL AND BIOPHYSICAL RESEARCH COMMUNICATIONS, ACADEMIC PRESS INC. ORLANDO, FL, US, vol. 313, no. 1, 2 January 2004 (2004-01-02), pages 163-170, XP004479132 ISSN: 0006-291X

## Description

### Field of the Invention

The present invention relates to an immunoassay kit, especially to a **neuroglobin enzyme-linked immunosorbent assay kit** (NGB-ELISA-kit) for detecting the neuroglobin and a use thereof.

### Background of the Invention

Neuroglobin (NGB) is the third kind of oxygen-carrying protein except for hemoglobin and myoglobin. The NGB protein is specially expressed in nervous system and distributed extensively in the brain. Similar to myoglobin, the NGB protein can reversibly bind oxygen with very high binding affinity. It is well known that the concentration of myoglobin in the serum is much lower for detection in normal individuals, so when the cardiac muscles and skeletal muscles are injured, the myoglobin will be released into the blood from the injured cells, resulting in a significant increase of the myoglobin protein in the serum. Accordingly, the concentration of myoglobin in the serum (serum-myoglobin) has been considered to be a specific and sensitive biomarker for some important diseases such as myocardial infarction and renal failure, *etc.* Interestingly, the feature of NGB and MGB are very similar to each other. For example, there are 151 amino acids (aa) in the NGB protein, and 154 aa in the MGB protein. The molecular weight for MGB and NGB are both 17 kD. Because that the functions of NGB and MGB are so similar (i.e., MGB is responsible for the oxygen supply of cardiac muscle and skeletal muscle, while NGB is responsible for the oxygen supply of brain), and both of them can be released into the serum from the injured cells, it is reasonable to infer that the concentration of the NGB protein in the serum has potential value for the clinical diagnosis of the nervous system diseases such as Alzheimer's disease, stroke and other brain damages. Analogously, detection of the NGB protein in the blood is also of significant value for revealing the disease stage of the above-mentioned nervous system diseases.

Currently, the main method for detecting the NGB protein is the immunoblotting analysis. In detail, the users need to perform SDS-PAGE at first for the test sample, and then detect the NGB protein with an anti-NGB antibody, followed by the reaction using a second specific enzyme-labeled antibody and then exposing the signal of the band on the X film. Finally, the users need to scan the film into a computer to perform a further image analysis to estimate approximately the level of the NGB protein in the test sample. The most disadvantage of this method is that the exact concentration of the NGB protein cannot be obtained.

### Summary of the Invention

The object of the present invention is to provide a NGB-ELISA-kit for measuring the concentration of the NGB protein in various samples.

The NGB-ELISA-kit of the present invention comprises an anti-NGB monoclonal antibody, an anti-NGB polyclonal antibody, an enzyme-labeled antibody and standard NGB protein, wherein the anti-NGB monoclonal antibody is obtained by immunizing a mice with NGB antigen followed by cell fusion to produce a hybridoma cell and subsequently culturing the hybridoma cell, wherein the anti-NGB polyclonal antibody is obtained by immunizing an animal with NGB antigen and then harvesting from serum of the animal, and wherein the standard NGB protein is NGB antigen obtained by the following steps:
(1) constructing a prokaryotic expression vector containing a full length human NGB cDNA sequence;
(2) transforming the constructed prokaryotic expression vector into *E. coli* HB101 strain, and then screening positive clones;
(3) inducing the expression of NGB by thermal stimulation to obtain an expression product; and
(4) purifying the expression product by gel filtration firstly and then by anion exchange chromatography to obtain the NGB antigen.

More specifically, the prokaryotic expression vector is pBV220-rhNGB.

The conditions for culturing the positive clones are: inoculating the *E. coli* strain in LB culture medium containing 80-120 µg/ml of ampicillin and culturing for 2-3 h at 28-32°C, followed by culturing with shaking at 40-44°C to induce the expression of rhNGB.

In the kit according to the invention, the enzyme-labeled antibody is typically HRP-labeled goat-anti-rabbit immunoglobulin.

Generally, the NGB-ELISA-kit further comprises enzyme-labeling plate, standard NGB protein and color-developing reagent. In the present invention, color-developing reagent is TMB (3,3',5,5'-tetramethyl benzidine).

Another object of the present invention is to provide a use of the NGB-ELISA-kit.

The kit of the present invention has high specificity and high sensitivity. Thus, the kit can be used in helping the clinical diagnosis of senile dementia, cerebral infarction or traumatic brain injury.

### Brief Description of the Drawing

Fig. 1 is a diagram showing the electrophoresis figure of the amplified product of rhNGB obtained by the PCR technique;
Fig. 2 are diagrams showing the restriction map of the rhNGB-positive clones after identifying by PCR and restriction enzyme method, respectively;
Fig. 3 is a diagram showing the immunoblotting analysis of the obtained monoclonal antibody;
Fig. 4 are diagrams showing the identification of the sub-class of the obtained monoclonal antibodies;
Fig. 5 are diagrams showing the immunoblotting analysis of the obtained polyclonal antibody;
Fig. 6 is a diagram showing the distribution of the NGB proteins in the brain of an adult gerbil detected by the obtained polyclonal antibody;
Fig. 7 is a diagram showing standard curve for the NGB protein detected by the kit of the present invention;
Fig. 8 are diagrams showing the distribution of the NGB positive neurons in the hippocampus of gerbil;
Fig. 9 are diagrams showing the distribution of the NGB positive neurons in the brain 72 hrs after ischemia reperfusion injury;
Fig. 10 is a diagram showing the serological change of the NGB in the brain of gerbil 2 to 72 hrs after ischemia reperfusion injury.

### Detailed Description of the Invention

### Example 1. The preparation of the NGB-ELISA-kit

### I. Preparation of the NGB antigen

### 1. Construction of the E. coli strain of pBV220-rhNGB/HB101 1

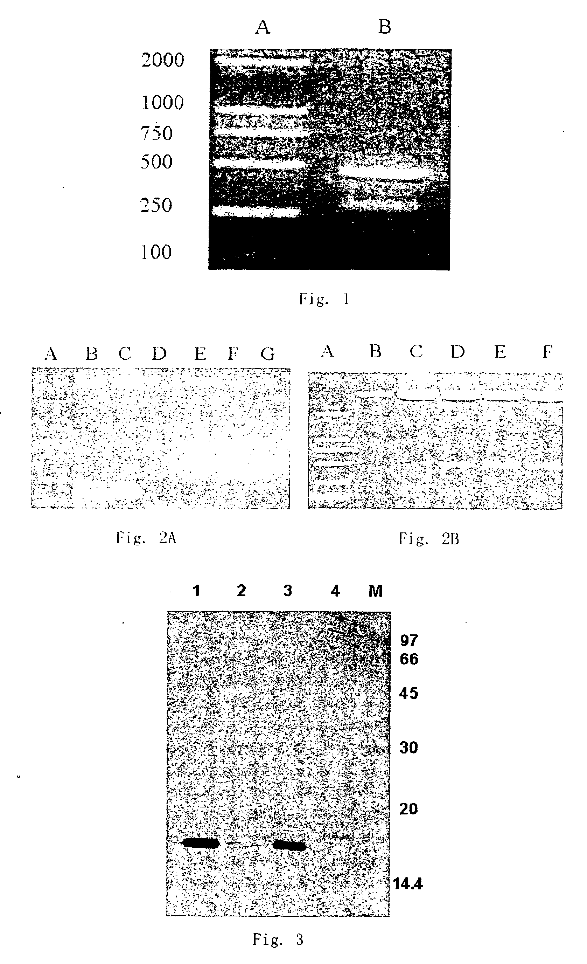

1). Synthesis of the primers: The NGB-specific primers pU and pD are designed and synthesized according to known sequence of a human NGB (hNGB) gene, wherein 5' end of 'pU' primer is incorporated with an *Eco*RI restriction site and a starting codon ATG, and 5' end of 'pD' primer is incorporated with a *Bam*HI restriction site. The primers were synthesized by Shanghai Boya Biotechnique Inc. The sequences of the primers are:
   pU: 5'-CCggAATTCATggAgCgCCCggAg-3'
   pD: 5'-ggTggATCCTTACTCgCCATCCCAgCCTCg-3'.
2) PCR amplification: The cDNA fragment of the human neuroglobin gene (namely Neurosurvivin (NSV)), which was obtained in our lab previously from the human fetal brain cDNA library by PCR technique) was used as template to perform the PCR amplification with the primers synthesized in the step 1) (Reference: Burmester T, Weich B, Reinhardt S, Hankeln T. A vertebrate globin expressed in the brain. Nature. 2000; 407 (6803): 520-523). The conditions for the PCR were: denaturation for 5 min at 95°C, followed by 30 cycles of 45 s at 94°C, 45 s at 60°C and 45 s at 72°C, and then elongation for 5 min at 72°C. After the reaction, 5 µl of the amplification product was detected by running 1.0% (w/v) agarose gel electrophoresis to check whether the coding region of the NGB gene was successfully amplified. The result is shown in Fig. 1, wherein lane A shows the DNA Marker DL2000, lane B shows the successfully amplified fragment of the NGB cDNA sequence (about 470 bp).
3) Construction of the expression plasmid: Both the highly-efficient expression vector pBV220 (available from Institute of Virology of Academy of Chinese Prevent Medical Science) and the purified PCR amplified products were digested by the restrictive enzyme *Eco*RI and *Bam*HI simultaneously and recovered by electrophoresis. Then the fragments were linked by T₄ DNA ligase to obtain the expressing plasmid pBV220-rhNGB.
4) Construction of the engineering bacteria: The plasmid pBV220-rhNGB was transformed into the competent *E. coli* HB101 cells (available from Beijing Baiweisheng Inc.) treated with CaCl₂. The transformants were selected and inoculated in 3 ml LB culture medium and cultured overnight at 30°C. The plasmid DNA was again extracted form the positive clone identified by PCR and was then identified by a further restriction analysis to confirm the construction. The results are shown in Fig. 2. In Fig. 2A, lane A represents the DNA Marker DL-2000, B-G indicates the identification of the recons by PCR. In Fig. 2B, lane A represents the DNA Marker DL-2000, lane B represents a pBV220 empty vector control, and C-F indicates the identification of the recons by double restrictive enzymes analysis with *Eco*RI and *Bam*HI.

The results suggest that there is a DNA fragment of about 470 bp in all recombinant plasmids after identification by PCR as well as *Eco*RI and *Bam*HI double restrictive analysis, which matches the expected NGB gene fragment (Neurosurvivin). The positive clone was named as pBV220-rhNGB/HB101 and was used as an engineering strain for expressing the rhNGB protein.

### 2. Activation of the genetically-modified engineering bacteria

5 µl of the bacteria stable strain stored at 4°C was used and inoculated at 5 ml LB culture medium containing 100 µg/ml ampicillin, followed by culturing while shaking at 200 rpm for 8 hrs at 30°C. 1% (v/v) of the cultures were then inoculated in the culture flask (culture medium was the same as before). The cultured strain was maintained for culturing while shaking at 200 rpm at 30°C for 16 hrs to obtain a seed culture.

### 3. Inducing the expression of rhNGB

The seed culture was inoculated at 3% (v/v) in a 150 ml LB culture medium containing 100 µg/ml ampicillin and cultured while shaking at 200 rpm for 2-3 hrs at 30°C. After the OD₆₀₀ reached at 0.3-0.4, the cultures were then transferred immediately into a water bath of 42°C and kept culturing while shaking for 5 hrs to induce the expression of rhNGB.

### 4. Collecting the bacteria, washing and freezing/thawing

The bacteria after induction were collected by centrifugation at 6000 rpm at 4°C for 15 min. A small amount of the bacteria were used to detect the expression of the rhNGB protein by SDS-PAGE analysis. The bacteria were washed for three times with TE buffer (25mM Tris-HCl, pH8.0, 1mM EDTA). After then, the bacteria were placed into liquid nitrogen for 5 min and then thawed in an ice bath. The freezing/thawing procedure was repeated for three times. 10-fold volume of TE buffer (25 mM Tris-HCl, pH8.0, 1mM EDTA) were then added to suspend the bacteria.

### 5. Lysis of the bacteria

The bacteria suspended with TE buffer (25 mM Tris-HCl, pH8.0, 1mM EDTA) was ultrasonicated (400 W) in an ice bath for 30s at a interval of 20s. The ultrasonication treatment was repeated for 15 times. The samples were then centrifuged at 12000 rpm for 20 min at 4°C. The supernatant was discarded and the inclusion was collected carefully.

### 6. Washing the inclusion

The inclusion was washed with Tris buffer (25mM Tris-HCl, pH8.0, 1M NaCl), centrifuged at 12000 rpm for 20 min at 4°C. The supernatant was discarded. The precipitation was washed twice with TE buffer (25mM Tris-HCl, pH8.0, 1mM EDTA) followed by a further centrifugation at 12000 rpm for 20 min at 4°C. The supernatant was discarded and the inclusion was collected.

### 7. Lysis of the inclusion

The inclusion was re-suspended in TE buffer containing 8 mol/L carbamide and 1% (v/v) mercaptoethanol and was ultrasonicated (400 W) for 30s at a interval of 20s. The ultrasonication treatment was repeated for 6 times. The resultant was boiled at 100°C for 5 min followed by a further centrifugation at 12000 rpm for 5 min at room temperature. Collecting the supernatant and discarding the precipitation.

### 8. Gel filtration

A Sephacryls-200 chromatographic column was used for further analysis, which was balanced at first with TE buffer containing 2 mol/L carbamide and 0.1% (v/v) mercaptoethanol. The sample prepared in step 7 was loaded on the chromatographic column, and was washed out by TE buffer containing 2 mol/L carbamide and 0.1 % (v/v) mercaptoethanol, and then collected the eluted fractions. After then, the eluents in each tube were transferred for SDS-PAGE analysis to detect the samples with more target proteins.

### 9. Anion exchange chromatography

The strong anion exchange chromatography column (Amersham Q Sepharose FF) was balanced at first with TE buffer containing 2 mol/L carbamide and 0.1% (v/v) mercaptoethanol. The samples which contained more target proteins were combined and loaded on the column, and were eluted gradually with the balancing buffer (TE buffer containing 2 mol/L carbamide and 0.1 % (v/v) mercaptoethanol) containing NaCl of different concentrations such as 0.05 M, 0.1 M, 0.15 M, 0.2 M, 0.5 M, and 0.7 M respectively. All of the eluenting peaks were collected. After then, the column was re-generated with the balancing buffer containing 1 M NaCl. At the same time, the samples of the eluents containing different NaCl concentrations were identified by SDS-PAGE analysis. As a result, it was found that the samples eluted by the balancing buffer containing 0.7 M NaCl contained the target proteins.

### 10. Renaturation and desalinization of the target protein rhNGB

A solvent resistant column Sephadex G-50 was balanced at first with TE buffer containing 0.1% (v/v) SDS. The samples containing target proteins were then loaded on the column, and then the column was washed by TE buffer containing 0.1% (v/v) SDS. All of the eluents were collected.

After analysis, the concentration of rhNGB in the culture medium was 1.2 g/L. The collected protein rhNGB was then identified and subjected to immunoblotting analysis.

### II. Identification of the recombinant full-length human NGB (rhNGB)

It is known that there are 456 bp in the coding region of the human neuroglobin gene Neurosurvivin (NSV), which can encode 151 amino acids (Reference: Burmester T, Weich B, Reinhardt S, Hankeln T. A vertebrate globin expressed in the brain. Nature. 2000; 407 (6803): 520-523)). The sequences of the nucleic acids and the amino acids were shown in SEQ ID No. 1 and SEQ ID No. 2, respectively.

After analyzing the rhNGB protein samples by mass spectrometer, the amino acids sequence was obtained as shown in SEQ ID No.3, wherein the amino acids matched with NSV fragment were 66 amino acids, which is about 43.7% of the full-length of the human neuroglobin protein (151 amino acids). Accordingly, it can be seen that the samples obtained by the method of the present invention are correct.

Furthermore, the amino acids sequence of the rhNGB protein was sequenced by direct sequencing using 491 protein sequence analyzer (ABI Compamy, USA, environment temperature was 20°C, relative humidity was 28%). The N-terminal of the sequence is M-E-R-P-E-P-E-L-I-R-Q-S-W-R-A-V, which completely match with the N-terminal of the human NGB protein. Thus, it can be seen that the samples obtained by the method of the present invention are completely correct.

### III. Preparation of the anti-NGB antibodies

### 1. Preparation of the monoclonal antibody

1) Experimental animal: Female BALB/c mice, 8-12 weeks age, 18-20 g weight (purchased from Animal Centre of Academy of Military Medical Sciences, People's Libration Army of China). The mouse-derived myeloma cells SP2/0 were purchased from the cell bank of Chinese Academy of Sciences, Shanghai.
2) Immunization: 100 µg, 125 µg and 150 µg of purified rhNGB were used as antigens and divided as three groups. In each group, the rhNGB was mixed with equal volume of freunds complete adjuvant respectively and emulsificated completely. The mixtures were then injected to three mice by intraperitoneal injection and subcutaneous injection with multiple sites. Three weeks later, same volumes of antigens were mixed with equal amounts of incomplete freunds adjuvant and were injected into the mice by intraperitoneal injection and subcutaneous injection with multiple sites. The immunization was repeated twice at a interval of four weeks. The potency was measured on 15-day after each immunization by collecting the serum. The mice with higher potency were intraperitoneally injected with 100 µg antigen to booster the immunization three days before cell fusion
3) Preparing the feeder cell: One day before the cell fusion, one normal Kunming mice was executed by cervical vertebra luxation and immersed in 70% ethanol for 5 min. After then, the skin of the abdomen was cut out at a clean bench and the peritoneum was exposed. 5 ml GKN solution (consisting of PBS supplemented with 11 mM d-glucose, 5.5 mM KCI, 137 mM NaCl, 25 mM Na₂HPO₄, and 5.5 mM NaH₂PO₄)was injected into the abdomen cavity by syringe. The ascites were then collected by repeatedly pressing the abdomen and then transferred to a centrifuging tube and centrifuged at 1000 rpm for 5 min. After discarding the supernatant, HAT culture medium was added to suspend the cells. The feeder cells were then transferred into a 96 well-plate and cultured in an incubator with 5% CO₂ at 37°C overnight.
4) Cell fusion: A mice with booster immunization was used for preparing the serum. In details, the eyes of the mice were removed to collect the blood and to prepare the serum which was stored at low temperature. Then, the mice was executed by cervical vertebra luxation and immersed in 70% ethanol for 5 min. After then, the spleen of the mice was taken out at a clean bench, which was further triturated to prepare cell suspension. The resultant was then centrifuged at 1000 rpm for 10 min at room temperature. After discarding the supernatant, the cells were washed with GKN solution twice, and re-suspended in GKN solution. 0.2 ml of the solution was taken for cell count under a microscope.
   The spleen cells were mixed with SP2/0 cells at a ratio of 10:1 in a 50 ml conical centrifuge tube, and centrifuged at 1500 rpm for 10 min at 37°C. The supernatant was discarded completely, and the cell precipitation was loosed by flicking the bottom of the tube. The centrifuge tube was placed in a 37°C water bath, and added dropwise with 1 ml 50% polyethylene glycol (PEG) solution with shaking for 1 min. 10 ml GKN solution (1 min for the first 2 ml, 2-3 min for the last 8 ml) was added after standing for 1 min to stop the cell fusion. After standing for another 1 min, the resultant was centrifuged at 1000 rpm for 10 min. The supernatant was discarded and hypoxanthine, aminopterin and thymidine (HAT) culture medium was added. Then stir with sucker gentlely to prepare a homogeneous suspension. The cells were inoculated into a 96 well-plate precoated with feeder cells and cultured in an incubator with 5% CO₂ at 37°C. The culture medium was changed every three days, and then was changed to hypoxanthine-thymidine (HT) culture medium 6 days later.
5) Screening for the positive clones: The modified dot-ELISA technique was used to screen for the positive hybridoma cells which can secrete anti-rhNGB antibody. In details, a nitrocellulose membrane (NC filter) was immersed in 0.01 mol/L PBS (pH7.4) for 15-30 min and then was dried by filter paper. The rhNGB protein was diluted with 0.01 mol/L PBS (pH7.4) to 50 µg/ml and was added dropwise onto the NC filter with 1 µl per dot and let it dry at room temperature. Then the membrane was placed into a blocking buffer for 30 min while shaking. The membrane was washed with washing buffer for 3×3 min. After dried by filter paper, the membrane was placed into the supernatant of cultured hybridoma cells (the supernatant of the SP2/0 cells was used as control) for 1 hr at room temperature. Then the membrane was washed with washing buffer for 3×3 min and immersed into the working solution of the second HRP-labeled antibody while shaking for 30 min at room temperature. The membrane was washed with washing buffer for 4×3 min. After then, the nitrocellulose membrane was placed into 3,3 - diaminobenzidine (DAB) solution and was shaked for 15 min. The resultant was washed with water for several minutes and then placed into distilled water to stop the reaction. The membrane was then allowed to dry at room temperature.
6) Sub-cloning of the hybridoma cells: The sub-cloning was performed with the limited dilution method. After counting the cells obtained in the plate with positive signals, the resultant with a concentration of 230 cells per 4.6 ml was re-suspended in a 96 well-plate containing 2-5×10⁴ feeder cells per well in HT culture medium. There are 36 wells containing 0.1 ml in each well. The left 1.0 ml was added into 4 ml HT culture medium and then added into the other 36 wells with 0.1 ml per well. The left 1.4 ml was added into 1.4 ml HT culture medium, and inoculated into the left 24 wells with 0.1 ml per well. Accordingly, there should be three groups with the ratio of the cell count of 5:1:0.5. After culturing in CO₂ incubator for four days, 100 µl HT culture medium was added into each well. Dulbecco's Modified Eagle's Medium (DMEM) culture medium was added on 9-day and then the proliferation of the cells was detected and the potency of the antibodies was determined. 1-2 well (wells) with higher antibody potency was (were) used for sub-cloning until the positive ratio is 100%. All of the other clones were collected and stored in the liquid nitrogen.
7) Monoclone strain: After screening and sub-cloning, four strains that could stably secrete anti-NGB hybridoma cells were obtained. They are named as 4G7, 4H1, 3E6 and 2E11, respectively.
8) Preparation of the ascites: The suspension of the monoclonal hybridoma cells obtained by sub-cloning was centrifuged at 1000 rpm for 5 min. Discard the supernatant and wash the cells with serum-free culture medium once. Add the serum-free culture medium again and then collect the supernatant for further use 3 days later. The mice were intraperitoneally injected with 0.5 ml liquid paraffin/mice. Seven days later, the mice were intraperitoneally injected with 5×10⁶/mouse of hybridoma cells suspension. 7-10 days later, the mice were observed to be swelled in the abdomen. The ascites were collected and stored at -20°C.

### 2. Preparation of the polyclonal antibody

Two young and healthy female rabbits were used to prepare the anti-NGB polyclonal antibodies. First, the rabbit was injected by 400 µg purified rhNGB protein (mixed previously with same volume of complete freunds adjuvant) intramuscularly injection and subcutaneously at back with multiple sites. Four weeks later, a same dosage (mixed previously with same volume of incomplete freunds adjuvant) was injected to booster the immunization. Two weeks later, the serum of the rabbit was collected by obtaining the blood from the carotid of the rabbit. The detailed procedure is described in 'Zhu Zhiping, Chen Xueqing. Common Experimental Methods in Immunology. Beijing: People's Surgeon Publishing House, 2000'.

### 3. Purification of the antibodies

The obtained mice ascites (monoclonal antibody) and the serum of rabbit (polyclonal antibody) were centrifuged, precipitated, desalted to obtain raw antibodies. Then, the antibodies were purified by a Protein G column to obtain the purified monoclonal antibody and polyclonal antibody. The detailed procedures are described in 'Zhu Zhiping, Chen Xueqing. Common Experimental Methods in Immunology. Beijing: People's Surgeon Publishing House, 2000'.

### 4. Quality control of the antibodies

### 1) Identification of the monoclonal antibody

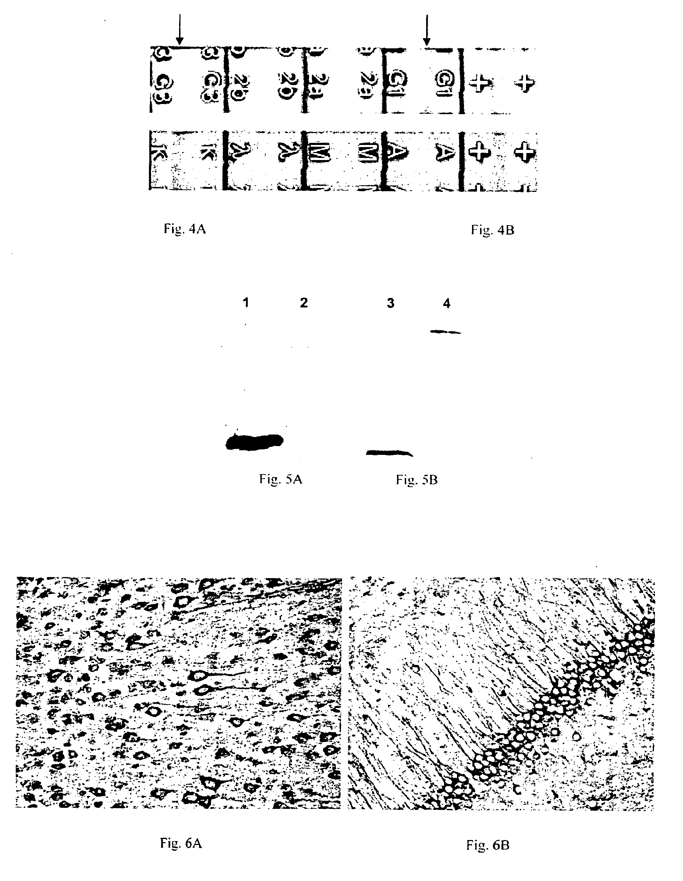

A. Identification of the specificity: The immunoblotting analysis was used to test the specificity of the monoclonal antibody. The results for identification of the antibody 2E11 were shown in Fig. 3, wherein lane 1 represents the supernatant of the lysis of the *E. coli* strain pBV220-rhNGB/HB101 that expresses the rhNGB protein; lane 2 represents the myoglobin as negative control; lane 3 represents the inclusion of *E. coli* strain pBV220-rhNGB/HB101; lane 4 represents the hemoglobin as negative control; lane M represents low molecular weight protein marker. It suggests that the monoclonal antibody has high specificity and recognizes the rhNGB protein without cross-reaction with other globins such as myoglobin and hemoglobin.
B. Identification of the sub-class of the antibodies: The antibody sub-class identifying kit (Roche, German) was used to detect the sub-class of the monoclonal antibody. Specifically, 150 µl serum-free culture medium diluted with 0.01 mol/L PBS (pH7.4) was added dropwise into a reaction tube and incubated for 30 sec at room temperature. Flick the tube transitorily to re-suspend the gel at the bottom completely. Place the sub-class identifying test paper into the reaction tube for 10 min and read the band to determine the sub-class of the monoclonal antibody. Results were shown in Fig. 4, which indicates that the heavy chain of the anti-NGB monoclonal antibody is IgG1 (Fig. 4A), while the light chain is κ chain (Fig. 4B).

### 2) Identification of the polyclonal antibody

The immunoblotting analysis was used for the identification of the polyclonal antibody. The abilities of the antibody to recognize rhNGB protein expressed either by prokaryotic cells or eukaryotic cells were tested.

Specifically, upon identification of the ability of the polyclonal antibody to recognize the rhNGB protein expressed by prokaryotic cells, we used the above-mentioned engineering vector pBV220-rhNGB/HB101 and the *E. coli* HB101 stains containing pBV220 empty vector in thermal induction. After the electrophoresis of the expression products, they were tested by immunoblotting analysis. The results were shown in Fig. 5A, wherein lane 1 suggests that the rabbit anti-rhNGB polyclonal antibody can recognize the rhNGB protein (about 17 kDa) contained in the expression product of the engineering vector pBV220-rhNGB/HB101; lane 2 represents the negative control which suggests that the rabbit anti-rhNGB polyclonal antibody cannot recognize any band in the expression product of the *E. coli* HB101 stains containing pBV220 empty vector.

When identifying the ability of the anti-NGB polyclonal antibody in recognization of the eukaryotic expressed NGB proteins, we used the NGB eukaryotic expression vector, pcDNA3.1N5/6×His/NGB (which is constructed by inserting the NGB gene sequence between the restriction sites, *BamH*I and *Xho*I, of the vector pcDNA3.1/V5/6×His (Invitrogen Inc.) using a forward primer of 5'-ggATCCgCATggAgCgCCCggAg-3' and a reverse primer of 5'-CTCgAgCTCgC CATCCCAgCCTCg-3', and eukaryoticly expressed NGB protein can be obtained after transforming the resulting recombinant vector into cells) and empty vector pcDNA3.1/V5/6×His to transfect the COS-7 cells, respectively. 48 hrs later, the cells were collected and all of the proteins in the cells were extracted for immunoblotting analysis. Results were shown in Fig. 5B, wherein lane 3 suggests that the rabbit anti-NGB polyclonal antibody can recognize the NGB proteins; lane 4 represents the positive control which indicates that the LacZ-6×His protein (the LacZ-6×His protein was obtained by transfecting the commercial available vectors pcDNA3.1/VS-His/*lac*Z (Invitrogen, U.S.)) can be detected by the anti-6×His monoclonal antibody. It suggests that the rabbit anti-NGB polyclonal antibody can recognize the NGB protein expressed in the eukaryotic cells.

The rabbit anti-NGB polyclonal antibody was further used to detect the distribution of the NGB protein in the brain of the gerbil. As shown in Fig. 6, it can be seen that the NGB-immunoreaction positive cells were widely distributed in the cerebral cortex (Fig. 6A) and the pyramidal cells and neurites of the hippocampus (Fig. 6B).

The results indicate that the anti-NGB polyclonal antibody prepared by the present invention can specifically recognize the NGB protein.

### IV. NGB-ELISA-kit

### 1. The following reagents were used in the NGB-ELISA-kit

1) Coating dilution buffer (0.85 M carbonate buffer, pH9.5): Na₂CO₃, 1.59 g; NaHCO₃, 2.93 g. Add double-distilled water to 1000 ml.
2) Washing buffer (PBS, pH7.4): KH₂PO₄, 0.2 g; KCI, 0.2 g; Na₂HPO₄·12H₂O, 2.9 g; NaCl, 8.0 g; Tween-20, 0.5 ml. Add double-distilled water to 1000 ml.
3) Sample dilution buffer: 0.1 g BSA, add the washing buffer to 100 ml.
4) Blocking buffer (1.5% casein, working concentration: 0.05%): Casein: 36 g; NaOH, 2.4 g; dissolved in 600 ml water. Add about 1800 ml PBS to fill the volume to 2400 ml.
5) Substrate buffer (Phosphate-citric acid buffer, pH5.0): 0.2M Na₂HPO₄ (28.4g/L) 25.7ml; 0.1M citric acid (19.2g/L) 24.3ml; add double-distilled water 50 ml.
6) TMB substrate dilution buffer: 3,3',5,5'-tetramethyl benzidine (TMB) (1mg/ml) 1.0 ml; substrate dilution buffer 10 ml; 30% H₂O₂ 7µl.
7) Stopping buffer (2M H₂SO₄): Add 21.7 ml concentrated sulfuric acid into 178.3 ml water dropwise.

The NGB-ELISA-kit of the present invention is made from above prepared anti-NGB monoclonal antibody and polyclonal antibody, together with the standard NGB protein and above reagents.

### 2. Protocols for using the NGB-ELISA-kit

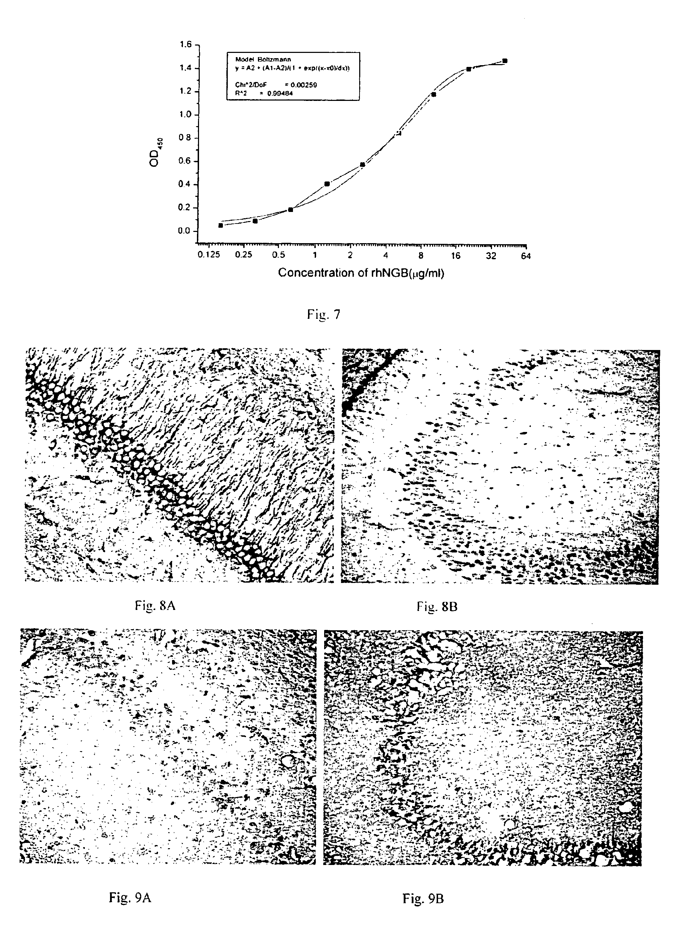

a) Coating: Dilute the anti-NGB monoclonal antibody with the coating buffer (0.85 M carbonate buffer, pH9.6) to 1:1000. Coat a 96-well enzyme-labeling plate with 100 µl per well at 4°C overnight.
b) Washing: Wash with the washing buffer PBST (0.01 M phosphate buffer (pH7.4) containing 0.05% Tween-20) for three times.
c) Blocking: Use the blocking buffer (0.05% casein) to block the well at room temperature for 4 hrs or overnight at 4°C, 125 µl per well.
d) Loading the samples: Set the wells for blank and negative control (double wells for each group). Add different concentration of the rhNGB standard into the wells (double well for each concentration) for obtaining the standard curve. Add the samples to be determined. For all of the well, the volume of the samples are 100 µl per well. Incubate in water bath at 37°C for 1 hr.
e) Washing: Wash with the washing buffer PBST (0.01 M phosphate buffer (pH7.4) containing 0.05% Tween-20) for three times.
f) Adding the polyclonal antibody: Dilute the polyclonal antibody to 1:1500 with PBS (0.01 M phosphate buffer, pH7.4), 100 µl per well. Incubate for 1 hr in water bath at 37°C.
g) Washing: Wash with the washing buffer PBST (0.01 M phosphate buffer (pH7.4) containing 0.05% Tween-20) for three times.
h) Adding second antibody: Dilute the HRP-labeled goat-anti-rabbit IgG to 1:5000 with the blocking buffer (1% casein), 100 µl per well. Incubate for 30 min in water bath at 37°C.
i) Washing: Wash with the washing buffer PBST (0.01 M phosphate buffer (pH7.4) containing 0.05% Tween-20) for three times.
j) Color developing: Develop with 3,3',5,5'-tetramethyl benzidine (TMB), 100 µl per well. Incubate for 10-20 min in water bath at 37°C.
k) Stopping: Stop the reaction with stopping buffer (2M H₂SO₄), 100 µl per well.
l) Detection: Detect the absorption of OD₄₅₀ by enzyme immunoassay instrument.
m) Obtaining the standard curve: Draw the standard curve, where the concentration of the standard protein was applied to be X axis, the OD₄₅₀ₙₘ was applied as Y axis. Result is shown in Fig. 7. It can be seen that the detectable region of the concentration of the NGB protein is 150 ng/ml to 18 µg/ml.
n) Calculation: The user can obtain the NGB concentration in the samples based on the standard curve. After multiplication by the dilution times, the exact concentration of NGB in the samples can be obtained.

### Example 2. The application of the NGB-ELISA-kit

The dynamic changes of the NGB protein in the serum after cerebral ischemia can be revealed by detecting the concentration of the NGB protein in serum of the animal model. The ischemia-reperfusion model of the gerbil brain was used and the serum was collected at 2 hrs, 8 hrs, 16 hrs, 24 hrs, 48 hrs and 72 hrs after reperfusion following 20 min of ischemia insults. First, the change of the tissues in the ischemia-reperfusion model was detected by using the anti-NGB polyclonal antibody of the present invention by immunohistochemical method, and the results are shown in Fig. 8 and Fig. 9. Wherein, Fig. 8A and 8B represent the normal distributions of the NGB-immunoreaction positive neurons in CA₁ and CA₃ region of the hippocampus of normal gerbil, Fig. 9A and 9B represent the distributions of NGB-immunoreaction positive neurons in CA₁ and CA₃ region of the hippocampus of gerbil after 72 hrs of reperfusion. After comparison, it can be seen that the neuron cells with ischemia-reperfusion injury are broken and a great numbers of them are dead.

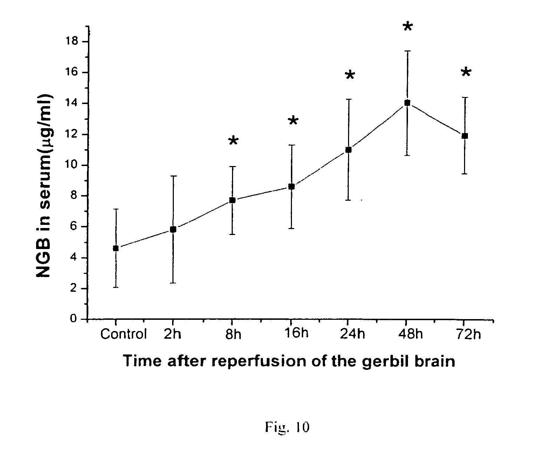

The serological dynamic change of NGB was detected by using the kit of the present invention, and it was compared with brain sections. The change of NGB in serum is shown in Table 1 and Fig. 10. It can be seen that after 8 hrs of ischemia-reperfusion injury of the brain of the gerbil, the concentration of NGB in the serum increased significantly (*P*<0.05) and gradually, which reached a peak at 48 hrs. The serological level of NGB is positively related to the degree of the brain injury in the ischemia-reperfusion model. Thus, it can be concluded that the detection of the serological level and the dynamic change of NGB can reveal the degree of the brain damage. This result clearly indicates that the NGB-ELISA-kit of the present invention can be used in studying the changing pattern of the NGB protein in the serum after cerebral infarction. At the same time, the result can be further used in evaluating the occurrence and the development of the cerebral infarction.

**Table 1. The change of NGB protein in the serum at different time after ischemia-reperfusion in the brain of gerbil (µg/ml)**

| Group | Numbers of test | Average | SD | SE | *P*-value |
|---|---|---|---|---|---|
| Control | 10 | 4.61 | 2.53 | 0.80 | |
| 2h | 6 | 5.81 | 3.50 | 1.41 | 0.436 |
| 8h | 6 | 7.69 | 2.19 | 0.89 | 0.027* |
| 16h | 5 | 8.59 | 2.70 | 1.21 | 0.011* |
| 24h | 6 | 10.97 | 3.27 | 1.33 | 0.001* |
| 48h | 6 | 14.02 | 3.38 | 1.38 | 0.000* |
| 72h | 5 | 11.94 | 2.47 | 1.10 | 0.000* |

| | | | | | |
|---|---|---|---|---|---|
| *: Compared to the control group, P<0.05 | | | | | |

Example 3. Detection of the NGB protein concentration in the serum samples of patient suffered from ischemia-related vascular disease and the rat ischemia-reperfusion injury model.

The NGB-ELISA-kit was used to detect the NGB protein concentration in the serum samples of patient suffered from ischemia-related vascular disease and the rat ischemia-reperfusion injury model. Detailed protocols were as follows:
a) Coating: Dilute the anti-NGB monoclonal antibody with the coating buffer (0.85 M carbonate buffer, pH9.6) to 1:1000. Coat a 96-well enzyme-labeling plate with 100 µl per well at 4°C for overnight.
b) Washing: Wash with the washing buffer PBST (0.01 M phosphate buffer (pH7.4) containing 0.05% Tween-20) for three times.
c) Blocking: Use the blocking buffer (0.05% casein) to treat the well at room temperature for 4 hrs or overnight at 4°C, 125 µl per well.
d) Loading the samples: Set the wells for blank and negative control (double wells for each group).
   (i) Add different concentration of the rhNGB protein into the wells (double well for each concentration) for obtaining the standard curve;
   (ii) Add the serum of the rat ischemia model (ischemia for 20 min followed by reperfusion for 48 hrs);
   (iii) Add the serum of patient suffered from ischemia-related vascular disease (purchased from the hospital No. 307, collected before the surgery, and the patient did not receive any treatment of special medicament. The blood routine examination is normal);
   (iv) For all of the well, the volume of the samples are 100 µl per well. Incubate in water bath at 37°C for 1 hr.
e) Washing: Wash with the washing buffer PBST (0.01 M phosphate buffer (pH7.4) containing 0.05% Tween-20) for three times.
f) Adding the polyclonal antibody: Dilute the polyclonal antibody to 1:1500 with PBS (0.01 M phosphate buffer, pH7.4), 100 µl per well. Incubate for 1 hr in water bath at 37°C.
g) Washing: Wash with the washing buffer PBST (0.01 M phosphate buffer (pH7.4) containing 0.05% Tween-20) for three times.
h) Adding second antibody: Dilute the HRP-labeled goat-anti-rabbit IgG to 1:5000 with the blocking buffer (1% casein), 100 µl per well. Incubate for 30 min in water bath at 37°C.
i) Washing: Wash with the washing buffer PBST (0.01 M phosphate buffer (pH7.4) containing 0.05% Tween-20) for three times.
j) Color developing: Develop with 3,3',5,5'-tetramethyl benzidine (TMB), 100 µl per well. Incubate for 10-20 min in water bath at 37°C.
k) Stopping: Stop the reaction with stopping buffer (2M H2SO4), 100 µl per well.
l) Detection: Detect the absorption of OD₄₅₀ by enzyme immunoassay instrument.
m) Obtaining the standard curve.
n) Based on the standard curve, the NGB concentration in the serum is 13 µg/ml in the patient, 11 µg/ml in the rat model of reperfusion for 48 hrs after ischemia insults for 20 min.

The result suggests that the NGB-ELISA-kit of the present invention can be used in detecting the exact NGB concentration in the serum of either patient or rat.

### Industrial Applicability

The NGB-ELISA-kit of the present patent is sensitive enough to detect the NGB protein concentration in various samples, and can be used as a potential guide line for clinical diagnosis of a lot of diseases related to neuron injury and degenerative diseases such as senile dementia, cerebral infarction or traumatic brain injury, and is helpful in tracing the development of the diseases.

### Sequence Listing

<160> 3
<210> 1
   <211> 456
   <212> DNA
   <213> Human neuroglobin
<400> 1
   atggagcgcc cggagcccga gctgatccgg cagagctggc gggcagtgag ccgcagcccg 60
   ctggagcacg gcaccgtcct gtttgccagg ctgtttgccc tggagcctga cctgctgccc 120
   ctcttccagt acaactgccg ccagttctcc agcccagagg actgtctctc ctcgcctgag 180
   ttcctggacc acatcaggaa ggtgatgctc gtgattgatg ctgcagtgac caatgtggaa 240
   gacctgtcct cactggagga gtaccttgcc agcctgggca ggaagcaccg ggcagtgggt 300
   gtgaagctca gctccttctc gacagtgggt gagtctctgc tctacatgct ggagaagtgt 360
   ctgggccctg ccttcacacc agccacacgg gctgcctgga gccaactcta cggggccgta 420
   gtgcaggcca tgagtcgagg ctgggatggc gagtaa 456
<210> 2
   <211> 151
   <212> PRT
   <213> Human neuroglobin
<400> 2
   Met Glu Arg Pro Glu Pro Glu Leu Ile Arg Gln Ser Trp Arg Ala Val
   1 5 10 15
   Ser Arg Ser Pro Leu Glu His Gly Thr Val Leu Phe Ala Arg Leu Phe
   20 25 30
   Ala Leu Glu Pro Asp Leu Leu Pro Leu Phe Gln Tyr Asn Cys Arg Gln
   35 40 45
   Phe Ser Ser Pro Glu Asp Cys Leu Ser Ser Pro Glu Phe Leu Asp His
   50 55 60
   Ile Arg Lys Val Met Leu Val Ile Asp Ala Ala Val Thr Asn Val Glu
   65 70 75 80
   Asp Leu Ser Ser Leu Glu Glu Tyr Leu Ala Ser Leu Gly Arg Lys His
   85 90 95
   Arg Ala Val Gly Val Lys Leu Ser Ser Phe Ser Thr Val Gly Glu Ser
   100 105 110
   Leu Leu Tyr Met Leu Glu Lys Cys Leu Gly Pro Ala Phe Thr Pro Ala
   115 120 125
   Thr Arg Ala Ala Trp Ser Gln Leu Tyr Gly Ala Val Val Gln Ala Met
   130 135 140
   Ser Arg Gly Trp Asp Gly Glu
   145 150
<210> 3
   <211> 151
   <212> PRT
   <213> Artificial sequence
<220>
   <223>
<400> 3
   Met Glu Arg Pro Glu Pro Glu Leu Ile Arg Gln Ser Trp Arg Ala Val
   1 5 10 15
   Ser Arg Ser Pro Leu Glu His Gly Thr Val Leu Phe Ala Arg Leu Phe
   20 25 30
   Ala Leu Glu Pro Asp Leu Leu Pro Leu Phe Gln Tyr Asn Cys Arg Gln
   35 40 45
   Phe Ser Ser Pro Glu Asp Cys Leu Ser Ser Pro Glu Phe Leu Asp His
   50 55 60
   Ile Arg Lys Val Met Leu Val Ile Asp Ala Ala Val Thr Asn Val Glu
   65 70 75 80
   Asp Leu Ser Ser Leu Glu Glu Tyr Leu Ala Ser Leu Gly Arg Lys His
   85 90 95
   Arg Ala Val Gly Val Lys Leu Ser Ser Phe Ser Thr Val Gly Glu Ser
   100 105 110
   Leu Leu Tyr Met Leu Glu Lys Cys Leu Gly Pro Ala Phe Thr Pro Ala
   115 120 125
   Thr Arg Ala Ala Trp Ser Gln Leu Tyr Gly Ala Val Val Gln Ala Met
   130 135 140
   Ser Arg Gly Trp Asp Gly Glu
   145 150

## Claims

1. An NGB-ELISA-kit comprising an anti-NGB monoclonal antibody, an anti-NGB polyclonal antibody, an enzyme-labeled antibody and standard NGB protein, wherein the anti-NGB monoclonal antibody is obtained by immunizing a mice with NGB antigen followed by cell fusion to produce a hybridoma cell and subsequently culturing the hybridoma cell, wherein the anti-NGB polyclonal antibody is obtained by immunizing an animal with NGB antigen and then harvesting from serum of the animal, and wherein the standard NGB protein is NGB antigen obtained by the following steps:
(1) constructing a prokaryotic expression vector containing a full length human NGB cDNA sequence;
(2) transforming the constructed prokaryotic expression vector into *E. coli* HB101 strain, and then screening positive clones;
(3) inducing the expression of NGB by thermal stimulation to obtain an expression product; and
(4) purifying the expression product by gel filtration firstly and then by anion exchange chromatography to obtain the NGB antigen.

2. The NGB-ELISA-kit according to claim 1, wherein the prokaryotic expression vector is pBV220-rhNGB.

3. The NGB-ELISA-kit according to claim 1, wherein the conditions for culturing the positive clones are: inoculating the *E. coli* strain in LB culture medium containing 80-120 µg/ml of ampicillin and culturing for 2-3 h at 28-32°C, followed by culturing with shaking at 40-44°C to induce the expression of rhNGB.

4. The NGB-ELISA-kit according to any of claims 1-3, wherein the enzyme-labeled antibody is HRP-labeled goat-anti-rabbit immunoglobulin.

5. The NGB-ELISA-kit according to any of claims 1-3, wherein the NGB-ELISA-kit further comprises enzyme-labeling plate and color-developing reagent.

6. The NGB-ELISA-kit according to claim 5, wherein the color-developing reagent is 3,3',5,5'-tetramethyl benzidine (TMB).

7. A use of the NGB-ELISA-kit according to claim 1 in helping the clinical diagnosis of senile dementia, cerebral infarction or traumatic brain injury.

## Patentansprüche

1. NGB-ELISA-Kit, der einen monoklonalen Anti-NGB-Antikörper, einen polyklonalen Anti-NGB-Antikörper, einen enzymmarkierten Antikörper und Standard-NGB-Protein umfasst, wobei der monoklonale Anti-NGB-Antikörper durch Immunisieren einer Maus mit NGB-Antigen und anschließender Zellfusion, wobei eine Hybridomzelle entsteht, und anschließendes Kultivieren der Hybridomzelle erhalten wird, wobei der polyklonale Anti-NGB-Antikörper durch Immunisieren eines Tiers mit NGB-Antigen und dann Ernten aus dem Serum des Tiers erhalten wird und wobei das Standard-NGB-Protein ein NGB-Antigen ist, das durch die folgenden Schritte erhalten wird:
(1) Konstruieren eines prokaryontischen Expressionsvektors, der eine vollständige humane NGB-cDNA-Sequenz enthält;
(2) Transformieren des *E.*-*coli*-HB101-Stamms mit dem konstruierten prokaryontischen Expressionsvektor und dann Selektieren von positiven Klonen;
(3) Induzieren der Expression von NGB durch thermische Stimulierung, wobei man ein Expressionsprodukt erhält; und
(4) Reinigen des Expressionsprodukts zuerst durch Gelfiltration und dann durch Anionenaustauschchromatographie, wobei man das NGB-Antigen erhält.

2. NGB-ELISA-Kit gemäß Anspruch 1, wobei es sich bei dem prokaryontischen Expressionsvektor um pBV220-rhNGB handelt.

3. NGB-ELISA-Kit gemäß Anspruch 1, wobei die Bedingungen zum Kultivieren der positiven Klone wie folgt sind: Einimpfen des *E*.-*coli*-Stamms in LB-Kulturmedium, das 80-120 µg/ml Ampicillin enthält, und Kultivieren während 2-3 h bei 28-32 °C und anschließendes Kultivieren unter Schütteln bei 40-44 °C, wobei die Expression von rhNGB induziert wird.

4. NGB-ELISA-Kit gemäß einem der Ansprüche 1 bis 3, wobei es sich bei dem enzymmarkierten Antikörper um HRP-markiertes Ziegen-Anti-Kaninchen-Immunglobulin handelt.

5. NGB-ELISA-Kit gemäß einem der Ansprüche 1 bis 3, wobei der NGB-ELISA-Kit weiterhin eine Enzymmarkierungsplatte und Farbentwicklungsreagens umfasst.

6. NGB-ELISA-Kit gemäß Anspruch 5, wobei es sich bei dem Farbentwicklungsreagens um 3,3',5,5'-Tetramethylbenzidin (TMB) handelt.

7. Verwendung des NGB-ELISA-Kits gemäß Anspruch 1 bei der Unterstützung der klinischen Diagnose von seniler Demenz, Hirninfarkt oder traumatischer Hirnverletzung.

## Revendications

1. Trousse de NGB-ELISA comprenant un anticorps monoclonal anti-NGB, un anticorps polyclonal anti-NGB, un anticorps marqué par une enzyme et une protéine NGB standard, dans laquelle l'anticorps monoclonal anti-NGB est obtenu par immunisation d'une souris avec un antigène de NGB, suivie d'une fusion cellulaire pour produire une cellule d'hybridome et ensuite, culture de la cellule d'hybridome, dans laquelle l'anticorps polyclonal anti-NGB est obtenu par immunisation d'un animal avec un antigène de NGB, puis récolte à partir du sérum de l'animal, et dans laquelle la protéine NGB standard est un antigène de NGB obtenu selon les étapes suivantes:
(1) construction d'un vecteur d'expression procaryote contenant une séquence d'ADNc de NGB humaine de taille complète;
(2) transformation du vecteur d'expression procaryote construit dans la souche HB101 de *E. coli,* et ensuite criblage des clones positifs;
(3) induction de l'expression de NGB par stimulation thermique pour obtenir un produit d'expression; et
(4) purification du produit d'expression tout d'abord par filtration sur gel, puis par chromatographie d'échange d'anions pour obtenir l'antigène de NGB.

2. Trousse de NGB-ELISA selon la revendication 1, dans laquelle le vecteur d'expression procaryote est le pBV220-rhNGB.

3. Trousse de NGB-ELISA selon la revendication 1, dans laquelle les conditions de culture des clones positifs sont: inoculation de la souche de *E. coli* dans un milieu de culture LB contenant 80-120 µg/ml d'ampicilline et culture pendant 2-3 h à 28-32°C, suivie d'une culture sous agitation à 40-44°C pour induire l'expression de rhNGB.

4. Trousse de NGB-ELISA selon l'une quelconque des revendications 1-3, dans laquelle l'anticorps marqué par une enzyme est l'immunoglobuline anti-lapin de chèvre marquée à la HRP.

5. Trousse de NGB-ELISA selon l'une quelconque des revendications 1-3, où la trousse de NGB-ELISA comprend en outre une plaque de marquage enzymatique et un réactif de développement de couleur.

6. Trousse de NGB-ELISA selon la revendication 5, dans laquelle le réactif de développement de couleur est la 3,3',5,5'-tétraméthylbenzidine (TMB).

7. Utilisation de la trousse de NGB-ELISA selon la revendication 1 pour aider au diagnostic clinique de la démence sénile, de l'infarctus cérébral ou de la lésion cérébrale traumatique.
